# FASCICULE DE BREVET EUROPEEN

(11) **EP 1 274 480 B1**
(45) Date de publication et mention de la délivrance du brevet: **05.07.2006**
(21) Numéro de dépôt: 01921506.0
(22) Date de dépôt: 06.04.2001
(51) Int. Cl.: A61M 25/01, A61B 1/005, B25J 18/06

(54) **DISPOSITIF DE POSITIONNEMENT, D'EXPLORATION ET/OU D'INTERVENTION NOTAMMENT DANS LE DOMAINE DE L'ENDOSCOPIE ET/OU DE LA CHIRURGIE MINI-INVASIVE**
VORRICHTUNG ZUR POSITIONIERUNG, UNTERSUCHUNG UND/ODER BEHANDLUNG, INSBESONDERE IM GEBIET DER ENDOSKOPIE UND/ODER MINIMAL INVASIVER CHIRURGIE
DEVICE FOR POSITIONING, EXPLORING AND/OR OPERATING IN PARTICULAR IN THE FIELD OF ENDOSCOPY AND/OR MINIMALLY INVASIVE SURGERY

(30) Priorité: 21.04.2000 FR 0005179; 21.04.2000 US 556673
(43) Date de publication de la demande: 15.01.2003
(73) Titulaire: UNIVERSITE PIERRE ET MARIE CURIE (PARIS VI), 75005 Paris (FR)
(72) Inventeur: FRANCOIS, Christian, F-75013 Paris (FR); BOUDGHENE, Frank, F-94120 Fontenay-sous-Bois (FR); JOLI, Pierre, F-77690 Montigny-sur-Loing (FR)
(74) Mandataire: Texier, Christian
(86) Numéro de dépôt international: PCT/FR2001/001050
(87) Numéro de publication internationale: WO 2001/080935

(56) Documents cités:
- WO-A-95/18311
- WO-A-96/35877
- US-A- 4 832 473
- US-A- 5 179 934
- US-A- 5 203 319

## Description

L'invention concerne les dispositifs de positionnement, d'exploration et/ou d'intervention notamment pour instruments médico-chirurgicaux tels que cathéters, endoscopes ou coeliolaparoscopes. Elle concerne aussi diverses industries pouvant employer ce type de dispositif dont la taille peut varier de quelques millimètres à plusieurs décimètres.

On connaît des instruments médicaux et chirurgicaux tels que des endoscopes ou des cathéters destinés à pénétrer dans des conduits ou des cavités du corps humain (vaisseaux, voies digestives, urinaires, etc.) à des fins d'observation ou d'intervention. Une des principales difficultés posées par ces instruments est qu'ils doivent pouvoir se déformer pour suivre la courbure du conduit ou de la cavité dans lesquels ils progressent ou pour emprunter l'une des deux voies d'un embranchement ou contourner un obstacle, tout en étant le moins agressifs possible pour les tissus. Le problème se pose par exemple dans le cas où on cherche à traiter un anévrisme par la pose d'une prothèse aortique. On connaît à cette fin des instruments dont la déformation est effectuée au moyen de câbles internes tirés ou poussés de façon à courber l'instrument dans un sens ou dans l'autre. C'est par exemple le cas dans le document EP-0 788 807. Toutefois, les instruments courbés par des tractions sur des câbles ne permettent pas d'obtenir des courbures très prononcées, ce qui peut compliquer la progression. De plus, ces instruments manquent souvent de souplesse et sont trop rigides. Le document « A Micro Robotic System for Colonoscopy, Proceedings of the 1997 IEEE International Conference on Robotics and Automation, Albuquerque, New Mexico, April 1997 » présente un instrument dont le corps est formé avec un soufflet lui permettant d'être à volonté allongé ou rétréci, et susceptible de progresser comme un ver dans le conduit. Toutefois, ce dispositif ne permet pas lui non plus de donner à l'instrument une courbure prononcée prédéterminée.

Dans un autre contexte, de nombreuses applications industrielles nécessitent de suppléer au manque de précision des machines ou des robots par un dispositif de repositionnement fin d'une pièce embarquée ou d'un outil terminal. Il s'agit par exemple soit d'aligner les repères de deux pièces à emboîter l'une dans l'autre (repositionnement en cours d'insertion), soit de minimiser les efforts de contact entre l'outil et une pièce à ouvrager (ébavurage par exemple).

Trois types de systèmes sont utilisés dans ce but :
- des dispositifs mécaniques traditionnels de précision combinant des axes de translation et de rotation dont les mouvements sont pilotés à partir d'informations de localisation relative délivrées par des détecteurs de position externes. On peut citer en exemple le recalage en cours de trajectoire d'un faisceau laser lors du soudage de deux pièces entre elles. Dans ce cas, l'information sur l'écart de position entre le plan de joint à suivre et la position réelle du faisceau laser résultant de la trajectoire nominale programmée est fournie par une caméra située en amont du faisceau ;
- des dispositifs compliants passifs. Il s'agit de systèmes mécaniques élastiques qui se déforment sous l'action des efforts extérieurs de contact entre l'outil ou la pièce embarquée et la pièce à usiner ou la pièce réceptrice. La disposition des éléments élastiques déformables et leur raideur sont judicieusement choisies de façon à ce que les mouvements de l'organe terminal (outil ou pièce embarquée) minimisent les efforts de contact dans toutes les directions le long de la trajectoire nominale programmée ; et
- des dispositifs compliants actifs. Il s'agit de systèmes mécaniques équipés d'actionneurs internes qui commandent les mouvements de l'organe terminal en fonction des valeurs des efforts de contact fournies par des capteurs de force intégrés au système. Là encore, il s'agit de minimiser les efforts de contact le long de la trajectoire programmée.

Dans ces domaines aussi, on recherche un dispositif de positionnement, d'exploration et/ou d'intervention pouvant concilier courbure commandée importante et souplesse.

Un but de l'invention est de fournir un dispositif de positionnement, d'exploration et/ou d'intervention pouvant être commandé pour obtenir des courbures prononcées tout en pouvant présenter une souplesse substantielle.

En vue de la réalisation de ce but, on prévoit selon l'invention un dispositif de positionnement, d'intervention et/ou d'exploration comportant deux embases et au moins un câble s'étendant de l'une à l'autre des embases, le dispositif comportant au moins deux soufflets directement fixés chacun aux deux embases et des moyens pour modifier une pression de fluide dans chacun des soufflets indépendamment du ou des autres soufflets.

Ainsi, les deux soufflets permettent de modifier la longueur du dispositif en modifiant la pression dans chacun des soufflets et en conservant les pressions égales entre les soufflets à chaque instant. On peut donc allonger ou rétrécir le dispositif. De plus, on peut rendre les pressions dans les soufflets différentes l'une de l'autre afin de courber le dispositif. On peut en outre combiner ces deux types de mouvement. On constate en pratique que les soufflets permettent d'obtenir des courbures du dispositif plus accentuées que dans les dispositifs classiques à commande uniquement par câble. Cela est notamment dû au fait que la courbure peut résulter conjointement d'une surpression dans un soufflet et d'une dépression dans un autre soufflet, alors que les câbles des dispositifs connus travaillent essentiellement en traction. Par ailleurs, comme on le voit, la déformation du dispositif est obtenue en jouant sur l'existence et l'amplitude d'un différentiel de pression, c'est-à-dire par la commande d'une pression relative. Mais en outre, le dispositif permet de choisir les pressions absolues régnant dans les soufflets afin de varier la souplesse ou la rigidité du dispositif. Ainsi, le travail avec des pressions élevées rendra le dispositif rigide face à des sollicitations externes. Au contraire, le travail avec des pressions basses rendra le dispositif relativement souple, déformable et peu agressif à l'égard de son environnement, avantage précieux lors d'une application dans le domaine médical. La commande de la pression permet de faire varier à volonté la rigidité du dispositif. On peut notamment choisir de donner au dispositif une raideur maximale ou au contraire une sensibilité maximale. Pour ces raisons, un tel dispositif de positionnement, d'exploration et/ou d'intervention est particulièrement avantageux dans le domaine médical (chirurgie,...) ainsi que dans de nombreuses industries.

De plus, la courbure du dispositif est ainsi commandée au choix par les soufflets et/ou par un ou plusieurs câbles.

Avantageusement, le dispositif comporte au moins trois soufflets fixés chacun aux deux embases.

Ainsi, on peut courber le dispositif dans n'importe quelle direction.

Avantageusement, le dispositif comporte au moins un câble s'étendant de l'une à l'autre des embases.

Avantageusement, le ou chaque câble est fixé à au moins l'une des embases et/ou est mobile par rapport à au moins l'une des embases.

Avantageusement, le ou chaque câble est décentré par rapport à un axe longitudinal du dispositif.

Avantageusement, le ou chaque câble s'étend à l'extérieur des soufflets.

Avantageusement, le dispositif comporte des tubes d'alimentation des soufflets en fluide, le ou chaque câble passant à l'extérieur des tubes.

Avantageusement, le ou chaque câble porte au moins une cale apte à venir en butée sur au moins une des embases lors de certaines déformations du dispositif.

Avantageusement, le ou chaque câble porte au moins deux cales aptes à venir en butée suivant deux sens opposés l'un à l'autre sur les deux embases.

Avantageusement, les câbles sont au nombre de deux par soufflet et disposés de part et d'autre du soufflet.

Avantageusement, le dispositif comporte au moins trois embases disposées suivant une succession, et pour chaque paire d'embases successives, au moins deux soufflets fixés chacun aux deux embases.

Le dispositif présente ainsi au moins deux étages dont les courbures peuvent se cumuler ou se combiner.

Avantageusement, le dispositif comporte pour chaque paire d'embases successives des moyens pour modifier une pression de fluide dans chacun des soufflets fixés aux embases de cette paire indépendamment du ou des autres soufflets fixés aux embases de cette paire.

Avantageusement, les moyens pour modifier la pression sont aptes à modifier la pression dans chacun des soufflets fixés aux embases de l'une des paires d'embases indépendamment des soufflets fixés aux embases de l'autre paire d'embases ou d'une autre parmi les paires d'embases.

Avantageusement, les deux soufflets de l'une des paires d'embases sont en communication de fluide avec les soufflets respectifs de l'autre paire d'embases ou d'une autre parmi les paires d'embases.

Avantageusement, le dispositif comporte au moins une entretoise disposée pour interdire un rapprochement des deux embases ou de deux des embases au-delà d'une valeur prédéterminée.

Avantageusement, chaque soufflet est apte à s'allonger et à se rétrécir suivant une direction longitudinale des soufflets sous l'effet du fluide sans modification substantielle d'une dimension du soufflet perpendiculairement à la direction longitudinale.

Ainsi, les courbures dans deux étages peuvent être dans des plans différents ou bien être dans le même plan. Dans ce cas, elles peuvent s'étendre dans la même direction ou dans des directions opposées.

Avantageusement, le dispositif comporte une première gaine intérieure fixée à l'une proximale des embases et une deuxième gaine extérieure recevant les embases et apte à coulisser sur la première gaine intérieure.

Ainsi, on peut modifier la longueur du dispositif, par action sur les soufflets, sans nuire à l'étanchéité entre l'extérieur et l'intérieur du dispositif.

Avantageusement, le fluide est un liquide.

Avantageusement, on peut combiner les variations de pression du liquide ou de tension du câble pour modifier la raideur et l'orientation du dispositif.

Avantageusement, le dispositif présente un espace longitudinal libre d'une extrémité à l'autre du dispositif.

Cet espace peut recevoir des éléments d'intervention, d'observation, etc.

Avantageusement, l'espace longitudinal central libre permet le passage d'instruments ou de guides d'onde.

On prévoit également selon l'invention un instrument médico-chirurgical, comportant un dispositif de positionnement, d'exploration et/ou d'intervention selon l'invention.

Avantageusement, il s'agit d'un cathéter.

Avantageusement, il s'agit d'un endoscope.

D'autres caractéristiques et avantages de l'invention apparaîtront encore dans la description suivante de deux modes préférés de réalisation et de variantes donnés à titre d'exemples non limitatifs. Aux dessins annexés :
- les figures 1 et 2 sont deux vues en perspective d'un dispositif de positionnement selon un premier mode de réalisation de l'invention ;
- la figure 3 est une vue en perspective avec arrachement partiel du dispositif de la figure 1 ;
- la figure 4 est une vue en coupe axiale d'un dispositif de positionnement selon un deuxième mode de réalisation de l'invention ;
- les figures 5 et 6 montrent la déformation en courbure respectivement d'un et de deux étages des dispositifs des figures précédentes,
- la figure 7 est une vue d'une embase d'un dispositif selon un troisième mode de réalisation de l'invention ;
- la figure 8 est une vue en perspective du dispositif du troisième mode de réalisation ; et
- la figure 9 est une vue analogue à la figure 7 illustrant un quatrième mode de réalisation de l'invention.

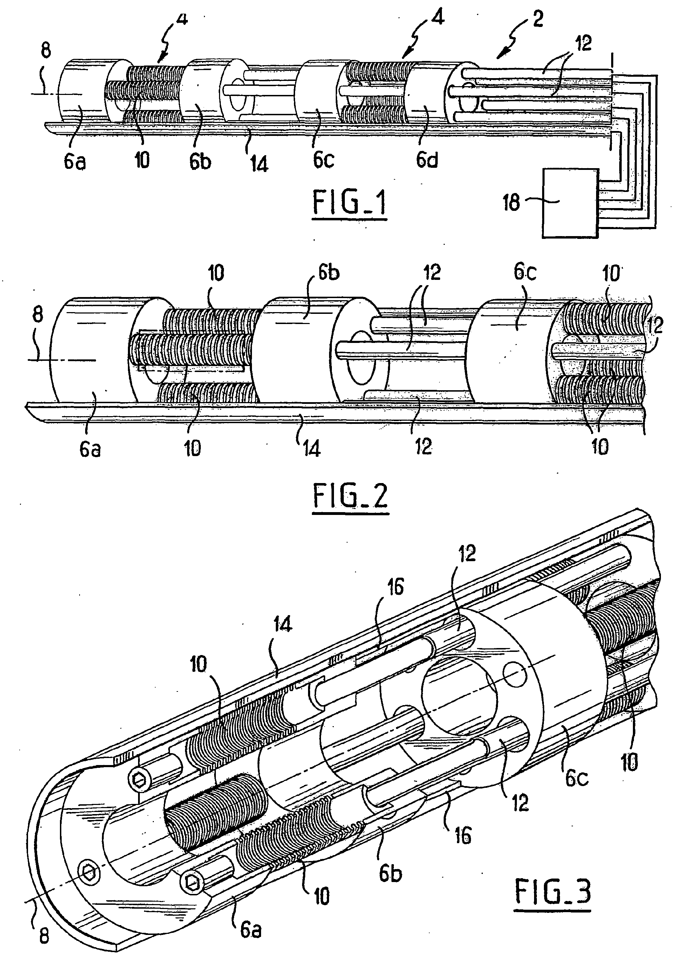

On va décrire en référence aux figures 1 à 3 un premier mode de réalisation de l'invention. Le dispositif de positionnement 2 comporte ici deux modules ou étages 4. Chaque étage 4 comprend deux embases 6 de forme cylindrique pleine mais percée de conduits comme on le verra dans la suite. Les quatre embases 6 sont identiques et disposées successivement avec leur axe 8 commun. On référence 6a, 6b, 6c et 6d les embases successives. L'embase 6a est l'embase distale correspondant à l'extrémité libre du dispositif. L'embase 6d est l'embase proximale et est la plus proche des moyens de commande et d'alimentation en fluide 18 du dispositif.

Les embases 6a et 6b forment une paire, ainsi que les embases 6c et 6d. A chacune de ces deux paires sont associés plusieurs soufflets 10 ici au nombre de trois pour chaque paire. Les six soufflets 10 sont identiques entre eux. Ils ont une forme générale cylindrique allongée parallèle à l'axe 8. Les trois soufflets de chaque paire d'embases sont régulièrement répartis autour de l'axe 8. Chaque soufflet est fixé par ses extrémités aux deux embases de la paire associée, aux faces d'extrémités axiales des embases. L'extrémité axiale distale de chaque soufflet est obturée.

Le dispositif comporte six conduits 12, un pour chaque soufflet 10, tous parallèles à l'axe 8. Trois des conduits aboutissent à l'embase proximale 6d sur sa face d'extrémité axiale proximale opposée aux soufflets. Ils traversent l'embase 6d et sont en communication de fluide, à l'intérieur de celle-ci, avec les soufflets respectifs à une extrémité axiale proximale de ceux-ci. Les trois autres conduits 12 traversent les deux embases 6d et 6c en étant régulièrement répartis autour de l'axe 8 et alternés avec les soufflets associés à cette paire. Ces trois conduits sont libres de coulisser à travers des orifices de ces deux embases 6c, 6d. Les trois conduits traversent ensuite l'espace entre les deux modules 4 puis arrivent à l'extrémité proximale de l'embase 6b.

La position radiale et l'écartement de ces conduits peuvent être choisies de façon à laisser un canal central de diamètre plus ou moins grand et un espace périphérique plus ou moins important permettant de loger un manchon annulaire replié (endoprothèse) et son éventuel dispositif de largage (ballon gonflable). A l'intérieur de l'embase 6b, les conduits sont mis en communication de fluide avec les trois soufflets 10 associés à cette embase. Il suit de cet agencement que les deux groupes de trois soufflets sont décalés l'un de l'autre d'un sixième de tour autour de l'axe 8, en vue axiale. Quant aux six conduits 12, ils peuvent être régulièrement répartis autour de l'axe 8. Il en est de même pour les trois conduits 12 associés à chaque paire. Chaque conduit 12 est en communication de fluide avec le soufflet 10 auquel il est associé et constitue avec celui-ci un ensemble étanche à l'égard des autres conduits et soufflets.

Tous ces éléments du dispositif sont enfermés dans une gaine souple 14 pouvant être reliée de façon étanche à l'embase distale 6a. L'écartement entre les deux embases 6b et 6c est préservé, quelle que soit la configuration du dispositif, au moyen d'entretoises 16 contiguës à la gaine 14 et prenant appui sur les extrémités axiales des embases 6b, 6c en regard. La longueur de ces entretoises peut être choisie de façon à obtenir un écartement prédéterminé des embases 6b et 6c. Alternativement, on pourra supprimer l'une de ces deux embases 6b, 6c afin que l'embase distale d'un module forme l'embase proximale de l'autre module.

La gaine 14 peut coulisser pour dégager l'espace entre les embases 6b et 6c, ce qui permet de larguer une endoprothèse repliée dans cet espace. De ce fait, la gaine 14 peut ne pas être reliée à l'embase distale 6a de façon étanche. Et les trois conduits d'alimentation situés entre les embases 6b et 6c peuvent être plus resserrés radialement que les trois autres conduits pour laisser un espace périphérique important entre les embases 6b et 6c.

Les soufflets sont élastiques et peuvent être allongés, rétrécis et /ou courbés. Le dispositif comporte des moyens 18 pour injecter un fluide sous pression, ici un liquide tel que du sérum physiologique, dans chacun des conduits 12 (et les soufflets associés). Ces moyens permettent de choisir la pression dans chacun des soufflets, indépendamment des autres soufflets. De tels moyens d'alimentation et de commande de la pression sont connus en soi et n'ont pas été représentés en détail. Sous une forme rudimentaire, il pourrait s'agir d'un jeu de six seringues anti-retour débrayables associées respectivement aux différents conduits 12. Sous une forme plus élaborée, il s'agira d'un distributeur apte à alimenter les différents conduits en fonction d'une commande de courbure globale donnée par un dispositif adapté, par exemple au moyen d'un clavier, d'une manette de commande de type joystick ou encore par commande vocale. Ces moyens de commande s'étendent à grande distance des embases et restent en dehors du corps dans le cas présent d'une exploration médico-chirurgicale.

En l'espèce, ils sont prévus pour pouvoir s'allonger et se rétrécir suivant leur direction longitudinale sous l'effet du fluide sans modification substantielle d'une dimension radiale des soufflets.

On va maintenant décrire comment on déforme un module 4. Pour l'allonger sans le courber, il suffit d'augmenter la pression dans chacun des soufflets 10 avec une pression instantanée égale dans les trois soufflets. De même, on diminue la pression de façon égale dans les trois soufflets pour rétrécir le module. Pour courber le module, il suffit de donner une pression plus importante à ou aux soufflets 10₁ situés du côté opposé au centre de courbure O comme illustré à la figure 5. Ainsi, ces soufflets s'allongent pour être plus longs que le ou les autres soufflets 10₂, entraînant l'inclinaison relative des embases 6 et provoquant la courbure du dispositif. Bien entendu, ces deux déformations peuvent être composées au sein d'un même module pour faire varier en même temps la longueur et la courbure

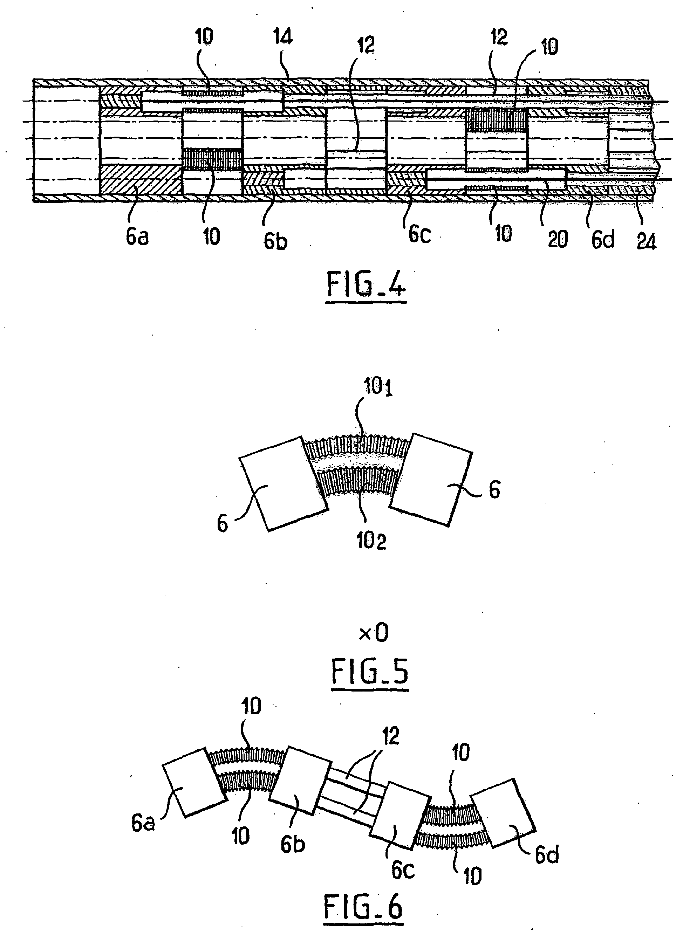

Les six conduits de commande 12 permettent de déformer chaque étage de façon totalement indépendante comme illustré à la figure 6 où les deux étages ont des courbures coplanaires mais en sens opposés. Précisément, les deux étages peuvent être commandés indépendamment l'un de l'autre. De plus, chacun des six soufflets peut être commandé indépendamment des autres. Lorsque les deux étages sont rétrécis ou allongés en même temps, ces déformations s'additionnent. Le dispositif selon l'invention possède une compliance active car on peut commander et contrôler sa déformation par un système de commande. Il possède également une compliance passive car il peut être déformé dans une certaine mesure sous l'effet de sollicitations extérieures. Ainsi, plus la pression dans les conduits sera élevée, plus le dispositif sera rigide. Au contraire, plus elle sera basse et plus le dispositif sera souple et compliant. Le dispositif présente ainsi une compliance mixte, à savoir passive et active. Les pressions de travail peuvent au choix être supérieures ou inférieures à la pression du milieu ambiant.

Les déformations obtenues par un tel dispositif sont plus importantes que dans le cas d'un dispositif commandé uniquement par des câbles, notamment en raison du fait que les câbles travaillent généralement en traction. Au contraire, le présent dispositif peut être commandé de sorte qu'au moins l'un des soufflets travaille en surpression alors qu'au moins un autre est en dépression de sorte que tous deux agissent pour courber l'étage dans le même sens.

Le dispositif présente un espace central libre au niveau de son axe 8, ininterrompu tout le long du dispositif. Cet espace s'étend entre les soufflets 10, entre les conduits 12 et est délimité par un orifice central des embases 6. Dans cet espace peuvent être logés au choix un instrument, un outil, une caméra, un tuyau d'injection de liquide, des câbles électriques, des fibres optiques, des rayons lumineux, des faisceaux lasers, un dispositif de vision, des instruments chirurgicaux, des aiguilles, des aiguilles à biopsie, des dispositifs d'agrafage, etc. (clamps, pinces, ciseau, ...)

Chaque module 4 peut être miniaturisé pour avoir une longueur inférieure à 2 cm et un diamètre inférieur à 5 mm.

Le mode de réalisation de la figure 4 est proche du précédent. Toutefois, il s'en distingue notamment par le fait que chaque soufflet 10 et son conduit 12 associé sont traversés, ici coaxialement, par un câble 20. Le câble est fixé à l'embase 6a ou 6c à laquelle est fixée l'extrémité distale obturée du soufflet. Les moyens de commande 18 permettent de tirer à volonté sur au moins un des câbles 20 sans agir sur les autres. La déformation du dispositif peut être obtenue par action sur la pression seule comme précédemment, par action sur les câbles seuls ou encore par action sur les deux en même temps, ce qui permet d'obtenir des déformations, en longueur ou en courbure particulièrement importantes et une raideur variable.

Ainsi, on peut rétrécir le module en diminuant la pression dans les soufflets. On peut également le rétrécir en exerçant une force de traction égale sur trois câbles de commande. Pour courber le module, on peut faire varier la pression d'un soufflet à l'autre du module comme expliqué en référence au premier mode. Mais aussi, les soufflets 10₂ peuvent être en même temps raccourcis par traction sur les câbles, ce qui augmente la courbure globale du dispositif. La combinaison de l'effet de la pression et de la traction du câble dans chaque soufflet permet de faire varier la raideur du dispositif.

Dans chacun de ces modes de réalisation, on pourra prévenir le risque de flambement des soufflets 10 en entourant chacun d'eux par une entretoise cylindrique 22 plus courte que la plus courte longueur prévue pour le soufflet, comme illustré en traits mixtes sur la figure 2.

Dans le mode de réalisation de la figure 4, le dispositif comporte deux gaines (ce qui est un aspect indépendant de la présence des câbles). En effet, il comporte une deuxième gaine 24 coaxiale à la première gaine 14, contiguë intérieurement à cette dernière, fixée à la face d'extrémité proximale de l'embase proximale 6d et s'étendant en direction des moyens de commande 18. Ces deux gaines peuvent coulisser l'une dans l'autre en fonction des mouvements de l'embase distale 6a par rapport à l'embase proximale 6d. On peut ainsi rétracter la gaine extérieure tout en maintenant en place par blocage longitudinal la gaine intérieure.

La gaine 14 (extérieure) peut coulisser sur la gaine 24 (intérieure). La gaine 24 sert à maintenir la position longitudinale de l'ensemble du dispositif puisque, lorsqu'elle est bloquée, l'embase 6d vient s'appuyer dessus et ne peut donc pas reculer pendant la rétraction de la gaine 14.

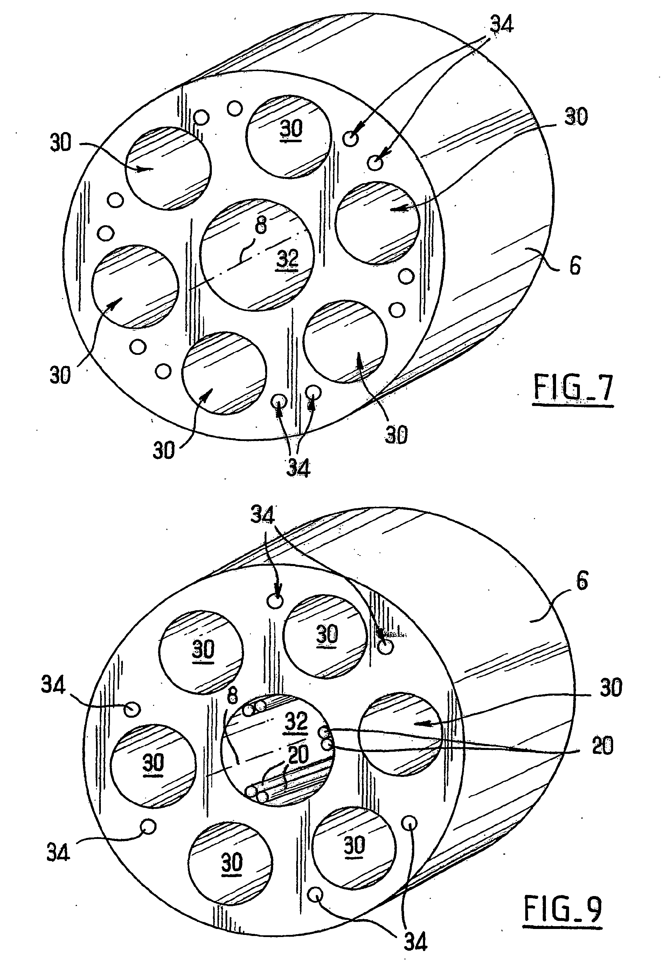

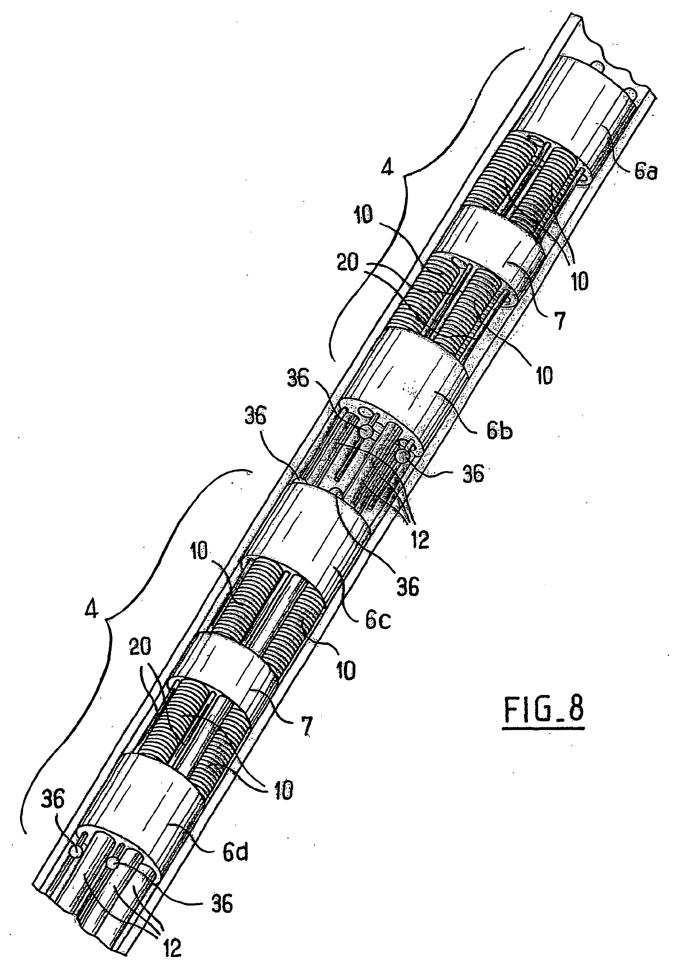

Un troisième mode de réalisation est illustré aux figures 7 et 8. Ici encore, le dispositif comporte deux étages 4, chaque étage comprenant deux embases principales 6. Chaque étage comporte en outre une embase supplémentaire et intermédiaire 7 s'étendant coaxialement entre les deux embases 6 et à mi-chemin de celles-ci. Dans chaque étage, chacun des 3 soufflets est remplacé par une paire de soufflets 10 s'étendant dans le prolongement l'un de l'autre, en communication de fluide l'un avec l'autre, et de part et d'autre de l'embase intermédiaire 7. Chacun des soufflets 10 est donc relié d'une part à une des embases principales 6 et d'autre part à l'embase intermédiaire 7.

Le remplacement de chaque soufflet du premier mode par deux soufflets disposés en série et commandés simultanément permet d'accroître l'amplitude de variation de longueur et de courbure de chaque étage.

De plus, l'embase intermédiaire 7 assure une fonction d'entretoise en évitant le flambement des soufflets qui pourrait se produire si les soufflets du premier ou du deuxième mode de réalisation avaient une grande longueur.

De plus, dans ce mode de réalisation, chaque étage est associé en propre en groupe à six câbles 20. L'ensemble du dispositif comporte donc douze câbles 20. En l'espèce, les câbles s'étendent hors des soufflets et des conduits 12. Chaque paire de soufflets est associée à deux câbles s'étendant de part et d'autre des soufflets comme on le voit aux figures 7 et 8. On a illustré à la figure 7 l'une des embases principales 6 de l'étage proximal 4, c'est-à-dire celui illustré en bas à gauche de la figure 8. L'embase présente six orifices principaux 30 régulièrement répartis autour de l'axe central 8 de l'embrase et du canal central 32. Les orifices 30 correspondent, en alternance spatiale, aux soufflets 10 de l'étage proximal et aux conduits 12 de l'étage distal.

L'embase présente douze orifices secondaires 34 associées par paire aux orifices principaux et s'étendant à proximité de ceux-ci. Les orifices secondaires servent au passage des câbles des deux étages. Naturellement, sur les embases de l'étage distal, le nombre d'orifices principaux et secondaires peut être réduit de moitié pour ne pas avoir d'orifice inoccupé.

Dans ce mode de réalisation, chaque câble 20 est mobile par rapport à toutes les embases 6, 7. Pour chaque embase principale à laquelle il est destiné à être relié effectivement, chaque câble 12 porte une cale 36 formée par exemple par un noeud sur le câble ou une pièce collée ou sondée au câble. Cette cale interdit le coulissement du câble dans l'orifice 34 de l'embase associée dans un certain sens au delà d'une certaine limite. Chaque câble porte deux cales disposées de sorte que le câble limite l'écartement entre les deux embases associées.

Chacune des cales les plus distales est utilisée lorsqu'on souhaite rétrécir le dispositif. Les deux cales de chaque câble servent en coopération à éviter la déformation plastique du soufflets lors d'une manoeuvre d'élongation.

On a illustré à la figure 9 un quatrième mode de réalisation qui constitue une variante du précédent. Cette fois, chaque embase porte six orifices secondaires 34 et non plus douze. Les six orifices sont disposés en trois paires autour des trois orifices principaux 30 associées à l'étage proximal. Ils servent encore au passage des six câbles associés. Les six câbles associés à l'étage distal passent cette fois dans le canal central 32 au niveau de l'étage proximal. Ils occupent les six orifices secondaires au niveau de l'étage distal.

Les embases peuvent être fabriquées en matériau métallique, plastique ou composite. Les soufflets peuvent être fabriqués en matériau métallique, plastique ou composite. Les soufflets peuvent être solidarisés aux embases de façon étanche, par collage, brasage, soudage, vissage, rivetage,.... Il en est de même, pour les tuyaux d'alimentation en fluide sous pression, qui doivent être souples et capables de résister aux pressions maximales que peuvent accepter les soufflets.

Les embases peuvent posséder également des évidements pour purger d'air les volumes sous pression (soufflets 10, tuyaux d'alimentation 12) en cas d'utilisation de fluides autres que l'air, et notamment de liquides. Ces évidements peuvent être obturés par des bouchons pour assurer l'étanchéité après la purge. Les embases peuvent présenter en outre des évidements pour accueillir des capteurs ou des systèmes d'accrochage d'outils de type pince, pince coupante, ciseaux, clamp, porte-aiguille, aiguille à biopsie, buses d'aspiration, etc.

Le dispositif pourra comporter des capteurs de position, par exemple des capteurs ultra sonores pour faciliter son guidage depuis l'extérieur par l'opérateur.

Les câbles peuvent être réalisés en alliage à mémoire de forme de façon à s'allonger ou se rétracter sous l'action de variations de température.

Les soufflets comprennent de préférence du nickel électro-déposé.

Le dispositif de positionnement selon l'invention pourra être conçu pour être à usage unique (jetable).

Du fait que les embases peuvent se déplacer suivant des mouvements de translation et de rotation les unes par rapport aux autres, on peut envisager de nombreuses applications.

### 1) Dans le domaine médico-chirurgical :

- réalisation d'endoscopes dont la courbure peut être modifiée de façon à suivre les méandres des voies naturelles à explorer, et dont l'extrémité orientable peut porter une microcaméra, un dispositif optique, diriger un rayon laser ou tout autre type d'onde ou un outil dans une direction donnée (dans les limites des débattements possibles) (aiguilles à biopsie, aiguilles d'injection, ciseaux, pinces, clamps, buse d'aspiration, bistouri électrique, etc.) ;
- réalisation de cathéters possédant les mêmes propriétés que les endoscopes décrits ci-dessus ;
- réalisation d'instruments de laparoscopie ou de coelioscopie équipés d'instruments chirurgicaux ;

### 2) Dans le domaine industriel :

Dans ce domaine, la taille du dispositif n'étant pas limitative, elle peut varier de quelques millimètres à plusieurs décimètres. Les applications sont :
- réalisation de dispositifs d'exploration et d'intervention dans des canalisations ;
- réalisation de doigts de préhension pour des pinces de robots ou de manipulateurs pour handicapés ;
- réalisation de pattes de robots marcheurs ou de véhicules à pattes. Les possibilités d'allongement et de rétraction des modules, combinées avec des mouvements transversaux dans deux directions perpendiculaires autorisent des déplacements longitudinaux et latéraux d'un véhicule à quatre pattes par exemple ;
- réalisation de dispositifs destinés à l'orientation dans l'espace de miroirs, de guides d'ondes, de fibres optiques, de faisceaux lumineux ou lasers ;
- réalisation d'articulations à raideur variable reliant deux solides entre eux ;
- réalisation d'un accouplement rotatif à inclinaison contrôlable. On peut imaginer par exemple un dispositif de forage comprenant un système de rotation et d'avance d'un outil de forage ou de perçage (cas des forages pétroliers ou autres). Si l'outil de forage (trépan, foret) est accouplé au dispositif de mise en rotation par un accouplement dont la déformation est programmée et synchronisée avec le mouvement de rotation de l'outil, on peut obtenir un forage en ligne courbe, résultat parfois recherché pour effectuer un forage horizontal à partir d'une plate-forme classique de forage vertical.

D'autres applications sont envisageables dans bien d'autres domaines, chaque fois qu'on désire orienter dans l'espace un solide par rapport à un autre.

Bien entendu, on pourra apporter à l'invention de nombreuses modifications sans sortir du cadre de celle-ci. Le nombre d'étages pourra être égal à un, deux, trois ou plus. Le nombre de soufflets par étage pourra être égal à deux, trois ou plus.

Dans les modes de réalisation qui viennent d'être décrits, on pourrait prévoir, bien que cela soit moins avantageux, que les soufflets des deux étages soient reliés en série au moins deux à deux de sorte que les conduits 12 sont au nombre de trois et commandent chacun deux soufflets, à savoir au moins un soufflet de chaque étage. Bien entendu, les deux soufflets ne peuvent plus alors être commandés indépendamment l'un de l'autre. Toutefois, on augmente l'amplitude de déformation axiale de chaque soufflet, et l'allongement de chaque module.

La paroi de chaque soufflet pourra avoir un profil de forme différente de la forme en zig-zag illustrée aux figures . Il pourra s'agir d'une paroi de forme cylindrique prévue pour présenter une élasticité en direction longitudinale mais pas en direction radiale.

Les câbles pourront être en tout matériau plastique notamment polymère tel que nylon, dacron, kevlar ou en métal.

## Revendications

1. Dispositif de positionnement, d'intervention et/ou d'exploration comportant deux embases (6a - 6d, 7) et au moins un câble (20) s'étendant de l'une à l'autre des embases (6a - 6d, 7), le ou chaque câble (20) étant mobile par rapport à au moins l'une des embases (6b, 6d), **caractérisé en ce qu'**il comporte au moins deux soufflets (10) directement fixés chacun aux deux embases et des moyens (12, 18) pour modifier une pression de fluide dans chacun des soufflets indépendamment du ou des autres soufflets.

2. Dispositif selon la revendication 1, **caractérisé en ce qu'**il comporte au moins trois soufflets (10) fixés chacun aux deux embases (6a - 6d, 7).

3. Dispositif selon l'une quelconque des revendications 1 à 2, **caractérisé en ce que** le ou chaque câble (20) est fixé à l'une des embases (6a-d, 7).

4. Dispositif selon l'une quelconque des revendications 1 à 3, **caractérisé en ce que** le ou chaque câble (20) est décentré par rapport à un axe longitudinal (8) du dispositif.

5. Dispositif selon l'une quelconque des revendications 1 à 4, **caractérisé en ce que** le ou chaque câble s'étend à l'extérieur des soufflets (10).

6. Dispositif selon l'une quelconque des revendications 1 à 5, **caractérisé en ce qu'**il comporte des tubes (12) d'alimentation des soufflets (10) en fluide, le ou chaque câble (20) passant à l'extérieur des tubes (12).

7. Dispositif selon l'une quelconque des revendications 1 à 6, **caractérisé en ce que** le ou chaque câble (20) porte au moins une cale (36) apte à venir en butée sur au moins une des embases lors de certaines déformations du dispositif.

8. Dispositif selon l'une quelconque des revendications 1 à 7, **caractérisé en ce que** le ou chaque câble (20) porte au moins deux cales (36) aptes à venir en butée suivant deux sens opposés l'un à l'autre sur les deux embases (6a-6d).

9. Dispositif selon l'une quelconque des revendications 1 à 8, **caractérisé en ce que** les câbles (20) sont au nombre de deux par soufflet et disposés de part et d'autre du soufflet.

10. Dispositif selon l'une quelconque des revendications 1 à 9, **caractérisé en ce qu'**il comporte au moins trois embases (6a - 6d) disposées suivant une succession, et pour chaque paire d'embases successives, au moins deux soufflets (10) fixés chacun aux deux embases.

11. Dispositif selon la revendication 10, **caractérisé en ce qu'**il comporte pour chaque paire d'embases successives des moyens (12, 18) pour modifier une pression de fluide dans chacun des soufflets (10) fixés aux embases de cette paire indépendamment du ou des autres soufflets fixés aux embases de cette paire.

12. Dispositif selon la revendication 10 ou 11, **caractérisé en ce que** les moyens pour modifier la pression sont aptes à modifier la pression dans chacun des soufflets fixés aux embases de l'une des paires d'embases, indépendamment des soufflets fixés aux embases de l'autre paire d'embases ou d'une autre parmi les paires d'embases.

13. Dispositif selon la revendication 10 ou 11, **caractérisé en ce que** les deux soufflets de l'une des paires d'embases sont en communication de fluide avec les soufflets respectifs de l'autre paire d'embases ou d'une autre parmi les paires d'embases.

14. Dispositif selon, l'une quelconque des revendications 1 à 13, **caractérisé en ce qu'**il comporte au moins une entretoise (16) disposée pour interdire un rapprochement des deux embases ou de deux des embases au-delà d'une valeur prédéterminée.

15. Dispositif selon l'une quelconque des revendications 1 à 14, **caractérisé en ce que** chaque soufflet est apte à s'allonger et à se rétrécir suivant une direction longitudinale des soufflets sous l'effet du fluide sans modification substantielle d'une dimension du soufflet perpendiculairement à la direction longitudinale.

16. Dispositif selon l'une quelconque des revendications 1 à 15, **caractérisé en ce qu'**il comporte une première gaine intérieure (24) fixée à l'une proximale (6d) des embases et une deuxième gaine extérieure (14) recevant les embases (6a-6d) et apte à coulisser sur la première gaine intérieure.

17. Dispositif selon l'une quelconque des revendications 1 à 16, **caractérisé en ce que** le fluide est un liquide.

18. Dispositif selon l'une quelconque des revendications 1 à 17, **caractérisé en ce qu'**il présente un espace longitudinal libre d'une extrémité à l'autre du dispositif.

19. Dispositif selon la revendication 18, **caractérisé en ce que** l'espace longitudinal libre est agencé pour permettre le passage d'instruments ou de guides d'ondes.

20. Instrument médico-chirurgical, **caractérisé en ce qu'**il comporte un dispositif de positionnement, d'exploration et/ou d'intervention selon l'une quelconque des revendications 1 à 19.

21. Instrument selon la revendication 20, **caractérisé en ce qu'**il s'agit d'un cathéter.

22. Instrument selon la revendication 20, **caractérisé en ce qu'**il s'agit d'un endoscope.

## Claims

1. A positioning, intervention, and/or exploration device having two endpieces (6a - 6d, 7) and at least one cable (20) extending from one endpiece to the other (6a - 6d, 7), the or each cable (20) being movable relative to at least one of the endpieces (6b, 6d), the device being **characterized in that** it comprises at least two bellows (10) each fixed directly to the two endpieces, and means (12, 18) for modifying the pressure of a fluid in each of the bellows independently of the other bellows.

2. A device according to claim 1, **characterized in that** it comprises at least three bellows (10) each fixed to the two endpieces (6a - 6d, 7).

3. A device according to claim 1 or 2, **characterized in that** the or each cable (20) is fixed to one of the endpieces (6a-d, 7).

4. A device according to any one of claims 1 to 3, **characterized in that** the or each cable (20) is off-center relative to the longitudinal axis (8) of the device.

5. A device according to any one of claims 1 to 4, **characterized in that** the or each cable extends outside the bellows (10).

6. A device according to any one of claims 1 to 5, **characterized in that** it includes feed tubes (12) for feeding the bellows (10) with fluid, the or each cable (20) passing outside the tubes (12).

7. A device according to any one of claims 1 to 6, **characterized in that** the or each cable (20) carries at least one block (36) suitable for coming into abutment against at least one of the endpieces during certain deformations of the device.

8. A device according to any one of claims 1 to 7, **characterized in that** the or each cable (20) carries at least two blocks (36) suitable for coming into abutment in two opposite directions against the two endpieces (6a-6d).

9. A device according to any one of claims 1 to 8, **characterized in that** the cables (20) are two in number per bellows and are disposed on either side of the bellows.

10. A device according to any one of claims 1 to 9, **characterized in that** it has at least three endpieces (6a - 6d) disposed in succession, and for each successive pair of endpieces, it has at least two bellows (10) each fixed to two endpieces.

11. A device according to claim 10, **characterized in that** for each successive pair of endpieces it includes means (12, 18) for modifying the pressure of a fluid in each of the bellows (10) fixed to the endpieces of the pair, and for doing so independently of the other bellows fixed to the endpieces of the pair.

12. A device according to claim 10 or 11, **characterized in that** the means for modifying the pressure are suitable for modifying the pressure in each of the bellows fixed to the endpieces of one of the pairs, and for doing so independently of the bellows fixed to the endpieces of the other pair or of another pair amongst the pairs of endpieces.

13. A device according to claim 10 or 11, **characterized in that** the two bellows of one of the pairs of endpieces are in fluid communication with the respective bellows of the other pair or of another pair amongst the pairs of endpieces.

14. A device according to any one of claims 1 to 13, **characterized in that** it includes at least one spacer (16) disposed to prevent two endpieces moving closer together or to prevent two of the endpieces moving closer together beyond a predetermined value.

15. A device according to any one of claims 1 to 14, **characterized in that** each bellows is capable of lengthening and of shortening along a longitudinal direction of the bellows under the effect of the fluid without substantial modification of the dimension of the bellows perpendicularly to the longitudinal direction.

16. A device according to any one of claims 1 to 15, **characterized in that** it has an inner first sheath (24) fixed to a proximal one (6d) of the endpieces, and an outer second sheath (14) receiving the endpieces (6a - 6d) and suitable for sliding in the inner first sheath.

17. A device according to any one of claims 1 to 16, **characterized in that** the fluid is a liquid.

18. A device according to any one of claims 1 to 17, **characterized in that** it has an empty longitudinal space extending from one end of the device to the other.

19. A device according to claim 18, **characterized in that** the empty longitudinal space is arranged to allow instruments or waveguides to be passed therealong.

20. A medico-surgical instrument, **characterized in that** it includes a positioning, exploration, and/or intervention device according to any one of claims 1 to 19.

21. An instrument according to claim 20, **characterized in that** it is a catheter.

22. An instrument according to claim 20, **characterized in that** it is an endoscope.

## Patentansprüche

1. Vorrichtung zur Positionierung, zum Eingriff und/oder zur Untersuchung, die zwei Basisteile (6a - 6d, 7) und wenigstens ein Kabel (20), das sich vom einen zum anderen der Basisteile (6a - 6d, 7) erstreckt, umfaßt, wobei das oder jedes Kabel (20) in Bezug auf wenigstens eines der Basisteile (6b, 6d) beweglich ist, **dadurch gekennzeichnet, daß** sie wenigstens zwei Faltenbalge (10), von denen jeder direkt an den beiden Basisteilen befestigt ist, und Mittel (12, 18) zur Veränderung eines Fluiddruckes in jedem der Faltenbalge unabhängig von dem oder den anderen Faltenbalgen umfaßt.

2. Vorrichtung nach Anspruch 1, **dadurch gekennzeichnet, daß** sie wenigstens drei Faltenbalge (10) umfaßt, von denen jeder an beiden Basisteilen (6a - 6d, 7) befestigt ist.

3. Vorrichtung nach einem der Ansprüche 1 bis 2, **dadurch gekennzeichnet, daß** das oder jedes Kabel (20) an einem der Basisteile (6a-d, 7) befestigt ist.

4. Vorrichtung nach einem der Ansprüche 1 bis 3, **dadurch gekennzeichnet, daß** das oder jedes Kabel (20) in Bezug auf eine Längsachse (8) der Vorrichtung dezentriert ist.

5. Vorrichtung nach einem der Ansprüche 1 bis 4, **dadurch gekennzeichnet, daß** das oder jedes Kabel sich außerhalb der Faltenbalge (10) erstreckt.

6. Vorrichtung nach einem der Ansprüche 1 bis 5, **dadurch gekennzeichnet, daß** sie Röhren (12) zur Versorgung der Faltenbalge (10) mit einem Fluid umfaßt, wobei das oder jedes Kabel (20) außerhalb der Röhren (12) verläuft.

7. Vorrichtung nach einem der Ansprüche 1 bis 6, **dadurch gekennzeichnet, daß** das oder jedes Kabel (20) wenigstens ein Anschlagstück (36) trägt, das dafür geeignet ist, bei bestimmten Deformationen der Vorrichtung auf wenigstens einem der Basisteile in Anschlag zu kommen.

8. Vorrichtung nach einem der Ansprüche 1 bis 7, **dadurch gekennzeichnet, daß** das oder jedes Kabel (20) wenigstens zwei Anschlagstücke (36) trägt, die dafür geeignet sind, in zwei zueinander entgegengesetzten Richtungen auf den beiden Basisteilen (6a - 6d) in Anschlag zu kommen.

9. Vorrichtung nach einem der Ansprüche 1 bis 8, **dadurch gekennzeichnet, daß** die Kabel (20) in einer Anzahl von zwei pro Faltenbalg vorliegen und zu beiden Seiten des Faltenbalgs angeordnet sind.

10. Vorrichtung nach einem der Ansprüche 1 bis 9, **dadurch gekennzeichnet, daß** sie folgendes umfaßt: wenigstens drei Basisteile (6a - 6d), die aufeinanderfolgend angeordnet sind, und für jedes Paar aufeinanderfolgender Basisteile wenigstens zwei Faltenbalge (10), von denen jeder an den beiden Basisteilen befestigt ist.

11. Vorrichtung nach Anspruch 10, **dadurch gekennzeichnet, daß** sie für jedes Paar aufeinanderfolgender Basisteile Mittel (12, 18) umfaßt, um einen Fluiddruck in jedem der Faltenbalge (10), die an den Basisteilen dieses Paares befestigt sind, unabhängig von dem oder den anderen an den Basisteilen dieses Paares befestigten Faltenbalgen zu verändern.

12. Vorrichtung nach Anspruch 10 oder 11, **dadurch gekennzeichnet, daß** die Mittel zur Druckveränderung dafür geeignet sind, den Druck in jedem der Faltenbalge zu verändern, die an den Basisteilen eines der Basisteilpaare befestigt sind, unabhängig von den Faltenbalgen, die an den Basisteilen des anderen Basisteilpaares oder eines weiteren unter den Basisteilpaaren befestigt sind.

13. Vorrichtung nach Anspruch 10 oder 11, **dadurch gekennzeichnet, daß** die zwei Faltenbalge des einen der Basisteilpaare in Fluidkommunikation mit den jeweiligen Faltenbalgen des anderen Basisteilpaares oder eines weiteren unter den Basisteilpaaren stehen.

14. Vorrichtung nach einem der Ansprüche 1 bis 13, **dadurch gekennzeichnet, daß** sie wenigstens ein Zwischenstück (16) umfaßt, das dafür angeordnet ist, eine Annäherung der beiden Basisteile oder von zweien der Basisteile über einen vorbestimmten Wert hinaus zu verhindern.

15. Vorrichtung nach einem der Ansprüche 1 bis 14, **dadurch gekennzeichnet, daß** jeder Faltenbalg dafür geeignet ist, sich in einer Längsrichtung der Faltenbalge unter der Wirkung des Fluids auszudehnen und zusammenzuziehen, und dies ohne eine wesentliche Veränderung einer Abmessung des Faltenbalgs senkrecht zur Längsrichtung.

16. Vorrichtung nach einem der Ansprüche 1 bis 15, **dadurch gekennzeichnet, daß** sie einen inneren ersten Mantel (24), der am einen proximalen (6d) der Basisteile befestigt ist, und einen äußeren zweiten Mantel (14), der die Basisteile (6a - 6d) aufnimmt und der dafür geeignet ist, auf dem inneren ersten Mantel zu gleiten, umfaßt.

17. Vorrichtung nach einem der Ansprüche 1 bis 16, **dadurch gekennzeichnet, daß** das Fluid eine Flüssigkeit ist.

18. Vorrichtung nach einem der Ansprüche 1 bis 17, **dadurch gekennzeichnet, daß** sie von einem Ende der Vorrichtung zum anderen einen längs verlaufenden freien Raum hat.

19. Vorrichtung nach Anspruch 18, **dadurch gekennzeichnet, daß** der längs verlaufende freie Raum dafür eingerichtet ist, die Durchführung von Instrumenten oder Wellenleitern zu gestatten.

20. Medizinisch-chirurgisches Instrument, **dadurch gekennzeichnet, daß** es eine Vorrichtung zur Positionierung, zum Eingriff und/oder zur Behandlung gemäß einem der Ansprüche 1 bis 19 umfaßt.

21. Instrument nach Anspruch 20, **dadurch gekennzeichnet, daß** es sich um einen Katheter handelt.

22. Instrument nach Anspruch 20, **dadurch gekennzeichnet, daß** es sich um ein Endoskop handelt.
